# EUROPEAN PATENT APPLICATION

(11) **EP 4 342 981 A1**
(43) Date of publication of application: **27.03.2024**
(21) Application number: 22804940.9
(22) Date of filing: 16.05.2022
(51) Int. Cl.: C12N 5/071

(54) **NOVEL METHOD FOR ISOLATING AND CULTURING DERMAL PAPILLA CELLS**

(30) Priority: 17.05.2021 KR 20210063593; 13.10.2021 KR 20210135996
(71) Applicant: Epi Biotech Co., Ltd., Incheon 21658 (KR)
(72) Inventor: ZHENG, Mei, Incheon 21658 (KR); CHOI, Nahyun, Incheon 22021 (KR); CHOI, Yong Jin, Suwon-si, Gyeonggi-do 16707 (KR); HWANG, Juyeong, Incheon 22006 (KR)
(74) Representative: Hoffmann Eitle
(86) International application number: PCT/KR2022/006996
(87) International publication number: WO 2022/245086

(57) **Abstract**

The present invention relates to a novel method for isolating and culturing dermal papilla cells. Particularly, in the isolation and culture method of the present invention, by culturing dermal papilla tissue isolated from the scalp under hypoxic conditions, dermal papilla cells separated from the dermal papilla tissue are rapidly attached to a culture plate, thus having the effect of shortening the period of establishing (harvesting) dermal papilla cells, and the isolated and cultured dermal papilla cells have immunocompatibility, and thus can be effectively used for the allogeneic transplantation of dermal papilla cells.

## Description

### Technical Field

The present invention relates to a novel method for isolating and culturing dermal papilla cells. Particularly, in the isolation and culture method of the present invention, by culturing dermal papilla tissue isolated from the scalp under hypoxic conditions, dermal papilla cells separated from the dermal papilla tissue are rapidly attached to a culture plate, thus having the effect of shortening the period of establishing (harvesting) dermal papilla cells, and the isolated and cultured dermal papilla cells have immunocompatibility.

### Background Art

Human hair is a collection of approximately 100,000 individual hairs, which are produced by hair follicles. Hair follicles serve as a reservoir of stem cells that can generate all the cell lines needed to reconstruct the hair follicle itself, the epithelium, and the sebaceous gland. There is a hair follicle bulb at the base of the hair follicle, and matrix cells that proliferate in the hair follicle bulb become hair. The hair follicle bulb contains dermal sheath cup (DSC) cells and dermal papilla cells (DPC). Dermal papilla cells are mesenchymally-derived fibroblasts located at the base of hair follicles and are key cells responsible for the development and growth of hair. U.S. Patent Registration No. 8227244 discloses a method for isolating dermal papilla cells from hair.

Hair grows, maintains, and falls out through a three-stage cycle: anagen, catagen, and telogen. It is known that during an anagen phase, which is the period during which hair grows, hair grows at an average of 0.3 mm per day in adults, grows about 1 cm per month, and usually lasts for 3 to 7 years. Generally, hair falls out after going through a catagen phase, which is a stage in which hair follicles shrink as overall apoptosis of hair follicle cells occurs for 10 to 14 days after the anagen phase, and a telogen phase, which is a phase in preparation for the next anagen that lasts an average of 3 months. The reason why the length of hair varies depending on the area is because the duration of the anagen phase, which is a unique characteristic of the hair follicle, is different for each area.

As such, since human hair has a certain hair growth cycle, it always maintains a certain number of hairs. However, as hair loss progresses, the dermal papilla cells present in the hair root become smaller, and as the dermal papilla cells become smaller, the thickness of the hair becomes thinner, and at the same time, the hair growth cycle becomes shorter. Therefore, as hair loss progresses, the hair becomes very thin and the hair growth cycle becomes shorter, causing it to grow a little and then fall out.

It is known that the causes of hair loss are not only genetic causes and the action of male hormones, but also a combination of factors such as severe physical and mental stress such as endocrine diseases, nutritional deficiencies, drug use, childbirth, fever, and surgery. Recently, not only male pattern hair loss, but also the number of women with hair loss is increasing due to increased stress due to changes in dietary life, social environment, and the like, and the age is also decreasing.

Currently, the most frequently used therapeutic agents for hair loss in Korea include finasteride (brand name: Propecia^{®}), dutasteride (brand name: Avodart^{®}), minoxidil (brand name: Minoxyl^{®} or Rogaine^{®}), and the like. However, the above therapeutic agents have side effects such as decreased libido, erectile dysfunction, loss of ability to drive and perform, itchiness at the application site, erythema, skin irritation, and eye irritation, and unwanted hair growth may be observed in other body parts other than the head. In addition, hair transplant surgery has recently been attempted for patients with severe hair loss, but the high cost and side effects after the surgery have been pointed out as limitations.

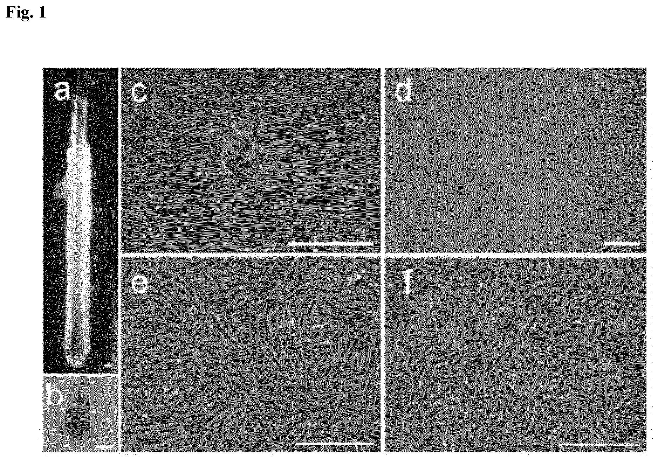

On the other hand, in addition to applying the drug directly to the skin, a technique of culturing and transplanting hair follicle cells in vitro has been attempted as a hair loss treatment method, but there are many difficulties in using dermal papilla cells as a hair loss therapeutic agent. First of all, it is difficult to dermal papilla cells isolate from the scalp, the culture conditions are strict, and it is difficult to culture a sufficient amount of cells. There are also limitations in that hair regeneration ability is significantly reduced when subcultured multiple times. The standard process of isolating dermal papilla cells can be summarized as follows (see Fig. 1, Archives of Dermatological Research 301(5):357-65): the step of isolating hair bulbs from the scalp tissue and isolating dermal papilla tissue where many dermal papilla cells exist; (a→b), the step of attaching dermal papilla cells from the dermal papilla tissue to a culture plate; (b→c), and the step of subculture (c→d), through which cell therapeutic agents or exosomes using the cells can be isolated from the harvested dermal papilla cells. Among these, it takes an average of 12 days or more for the dermal papilla cells from the dermal papilla tissue to be attached to the plate, resulting in an increase in the production period and production cost of the therapeutic agent using the dermal papilla cells. Therefore, in the development of cell therapeutic agents, it is very necessary to increase the attachment rate and decrease the attachment time of dermal papilla cells that are isolated from the dermal papilla tissue and attached to the plate.

Accordingly, the present inventors have discovered a novel method for isolating dermal papilla cells that can increase the attachment of dermal papilla cells to a culture plate and shorten the period of establishing dermal papilla cells through hypoxic (an oxygen saturation of 0.5 to 5%) culture in the process of establishing dermal papilla cells from the dermal papilla tissue in the isolation and culture of dermal papilla cells. Based on the above, the present inventors completed the present invention.

### Prior Art Document

### Patent Document

(Patent Document 1) U.S. Patent Registration No. 8227244

### Non-Patent Document

(Non-Patent Document 1) TGF-β2 and collagen play pivotal roles in the spheroid formation and antiaging of human dermal papilla cells. Kim H, Choi N, Kim DY, Kim SY, Song SY, Sung JH. Aging (Albany NY). 2021 Aug 17; 13(16): 19978 19995.

### Detailed Description of Invention

### Technical Problem

An object of the present invention is to provide a novel method for isolating and culturing dermal papilla cells. In addition, another object is to provide dermal papilla cells obtained by the isolation and culture method of the present invention, or a composition for preventing or treating hair loss, comprising a cell culture solution or exosomes obtained from the dermal papilla cells.

### Solution to Problem

The present invention provides a method for isolating and culturing dermal papilla cells, comprising culturing dermal papilla tissue isolated from the scalp under hypoxic conditions and attaching the dermal papilla cells to a culture plate.

The hypoxic conditions of the present invention may have an oxygen saturation of 0.5 to 5%, and preferably may have an oxygen saturation of 2%.

In the method for isolating and culturing dermal papilla cells of the present invention, the period required for attaching the dermal papilla cells to the culture plate may be shortened by 5 days or more compared to culturing the dermal papilla tissue under normoxic conditions.

The dermal papilla cells isolated and cultured according to the present invention may be immunocompatible dermal papilla cells, and the immunocompatible dermal papilla cells may inhibit the expression of any one or more mRNAs selected from the group consisting of HLA-A, HLA-B, CD55, IL-1RA, and CCL2 related to immune response.

The present invention provides dermal papilla cells obtained by a method for isolating and culturing dermal papilla cells, the method comprising culturing dermal papilla tissue isolated from the scalp under hypoxic conditions and attaching the dermal papilla cells to a culture plate, wherein the dermal papilla cells may be immunocompatible dermal papilla cells.

The present invention provides a pharmaceutical composition for preventing or treating hair loss, comprising dermal papilla cells obtained by a method for isolating and culturing dermal papilla cells, the method comprising culturing dermal papilla tissue isolated from the scalp under hypoxic conditions and attaching the dermal papilla cells to a culture plate.

The present invention provides a pharmaceutical composition for preventing or treating hair loss, comprising a cell culture solution or exosomes obtained from dermal papilla cells obtained by a method for isolating and culturing dermal papilla cells, the method comprising culturing dermal papilla tissue isolated from the scalp under hypoxic conditions and attaching the dermal papilla cells to a culture plate.

### Effects of Invention

According to the novel method for isolating and culturing dermal papilla cells of the present invention, in the process of isolating dermal papilla cells from the scalp tissue, when culturing the dermal papilla tissue under hypoxic conditions (an oxygen saturation of 0.5 to 5%, preferably 2%), dermal papilla cells separated from the dermal papilla tissue can be rapidly attached to a culture plate, and thus primary cells of the dermal papilla cells can be rapidly established. After isolation of primary cells of the dermal papilla cells, dermal papilla cells can be mass-produced through subculture, and the production period of cell therapeutic agents can be shortened. In addition, sufficient dermal papilla cells necessary for the process of treating hair loss can be obtained, and therefore can be effectively used for the treatment of hair loss.

In addition, the dermal papilla cells isolated and cultured according to the present invention have immunocompatibility, thereby reducing the immune response that may occur during the allogeneic transplantation of dermal papilla cells. Therefore, the dermal papilla cells can be used for the treatment of hair loss through transplantation of dermal papilla cells.

### Brief Description of Drawings

Fig. 1 shows the standard isolation process of dermal papilla cells from the scalp (Archives of Dermatological Research 301(5):357-65).
Fig. 2 shows the level of attachment to the culture plate of dermal papilla cells separated from the dermal papilla tissue under normoxic conditions (an oxygen saturation of 20%) and hypoxic conditions (an oxygen saturation of 2%).
Fig. 3 shows collagen genes related to cell attachment increased by hypoxic conditions (an oxygen saturation of 2%) through nucleotide sequence (NGS) and ontology analysis.
Fig. 4 shows the results obtained by comparing the mRNA expression related to the degradation of extracellular matrix and adhesion molecules in dermal papilla cells cultured under normoxic conditions (an oxygen saturation of 20%) and hypoxic conditions (an oxygen saturation of 2%).
Fig. 5 shows the mRNA expression level of genes related to immune response activity in dermal papilla cells cultured under normoxic conditions (an oxygen saturation of 20%) and hypoxic conditions (an oxygen saturation of 2%).

### Best Mode for Carrying out the Invention

Hereinafter, with reference to the accompanying drawings, embodiments and examples of the present disclosure will be described in detail so that those of ordinary skill in the art to which the present invention belongs can easily practice the present invention. However, the present disclosure may be implemented in various forms and is not limited to the embodiments and examples described herein.

Throughout the present specification, when a certain part "includes" a certain component, it means that other components may be further included, rather than excluding other components, unless otherwise stated.

The present invention provides a novel method for isolating and culturing dermal papilla cells.

In the present invention, "dermal papilla cells (DPC)" are mesenchymally-derived fibroblasts located at the base of hair follicles and are key cells responsible for the development and growth of hair.

As used herein, the term "hair loss" refers to the absence of hair in areas where hair should normally exist, regardless of the cause, and includes alopecia areata, hereditary androgenetic alopecia, telogen effluvium, traumatic alopecia, trichotillomania, pressure alopecia, anagen effluvium, alopecia pityroides, syphilitic alopecia, seborrheic alopecia, symptomatic alopecia, cicatricial alopecia, or congenital alopecia, but is not limited thereto.

As used herein, the term "treatment" refers to any action that beneficially changes hair loss symptoms, such as improving hair loss symptoms by delaying or stopping the hair loss process, or promoting hair growth and hair regrowth, such as lengthening or increasing the number of hairs by administering a composition.

As used herein, the term "flow cytometry" is a laser-based technology widely used to analyze the characteristics of cells or particles and can measure cell size, complexity, cell number, cell cycle, and the like. A laser is radiated to the fluid through which the cells to be measured are flowing, and the light passing through or scattered through the cells is detected and analyzed, thereby allowing only specific cell groups to be isolated from the population.

Hereinafter, the present invention will be described in more detail through the examples, but the following examples are for illustrative purposes only and are not intended to limit the scope of the present invention.

### [Example 1]

### Isolation of tissue

On the bottom of a sterilized 100 mm Petri dish, 20 mL of isolation/culture medium containing 4% FBS was filled to submerge the scalp tissue collected from the donor and stored to prevent the scalp tissue from drying out. After placing the tissue on the bottom of the Petri dish, it was cut into strips without overlapping hair follicles using a disposable scalpel, and the cut tissue strips were stored in a Petri dish containing the isolation medium where the tissue was stored. The cut tissue strip was placed on the bottom of a new Petri dish, and then hair follicles were isolated one by one using a disposable scalpel and stored in a Petri dish containing new medium.

### [Example 2]

### Isolation of dermal papilla tissue from hair bulb

A 150 mm Petri dish was placed on the stage of the dissection microscope, and one hair follicle isolated in Example 1 above was placed thereon. Thereafter, a drop of isolation medium was added using a 1 mL syringe to prevent the hair follicle from drying out. The border between the dermal sheath cup (DSC) and the upper dermal sheath (UDS) of the hair follicle was cut using a disposable scalpel. After adding a drop of isolation medium to the cut dermal sheath cup to prevent it from drying out, Two 1 mL syringes were used, one to fix the dermal sheath cup and the other to tap the bottom of the cup, which is the sharpest part of the dermal sheath cup, so that the cup was turned upside down. When elongated diamond-shaped or water drop-shaped dermal papilla tissue (DP) protruded from the inside of the inverted dermal sheath cup, only the dermal papilla tissue was cut out using a syringe.

### [Example 3]

### Attachment of dermal papilla cells from dermal papilla tissue to plate

The dermal papilla tissue (DP) isolated in Example 2 above was placed on the tip of a syringe needle and transferred to a 6 well plate (CellBind surface). Twelve pieces of the dermal papilla tissue were inoculated per well, placed in an incubator under normoxic conditions (5 % CO₂: 21 % O₂) and hypoxic conditions (5 % CO₂: 2 % O₂) at 37 °C without shaking the plate, and cultured for 5 to 10 days. Thereafter, the medium was changed twice (once every 2 to 3 days) until the confluency reached 60 % (p0). The culture medium until isolation (p0) and 5th passage (p5) was CellCor^{™} (XCELL therapeutics) or Follicle dermal papilla cell growth medium (Promaocell)/4 % Fetal bovine serum (Hyclone)/1 % penicillin streptomycin (Thermo Fisher scientific).

### [Example 4]

### Subculture of dermal papilla cells

When about 60% of the well were filled with dermal papilla cells separated from the dermal papilla tissue inoculated in Example 3 above, subculture was performed in a T75 flask. The medium in the 6 well plate was discarded and washed once with 1x DPBS, and then 0.5 mL of Accutase was added per well, and the mixture was reacted for 10 minutes in an incubator under each condition. Thereafter, 2 mL of isolation medium was dispensed per well, and then the entire mixture was collected in a 50 mL tube, and then the supernatant was removed by centrifugation for 3 minutes at room temperature and 1300 rpm. The cells remaining in the lower layer were suspended with 1 mL of culture medium, and then the number of cells was counted through trypan blue staining. The number of T75 flasks to be subcultured was determined according to the number of collected cells (2.5 × 10⁵ cells/flask). 15 mL of new culture medium was added to the T75 flask, the suspended cell solution was calculated and dispensed, and then the flask was carefully rotated at least three times to ensure that the cells were evenly mixed. Thereafter, they were cultured for 4 days in an incubator under normoxic conditions (5 % CO₂: 21 % O₂) and hypoxic conditions (5 % CO₂: 2 % O₂), and subcultured at a ratio of 1:5 from the 1st passage to the 5th passage ( p1 to p5) at 4-day intervals.

### [Experimental Example 1]

### Comparison of plate attachment levels of dermal papilla cells

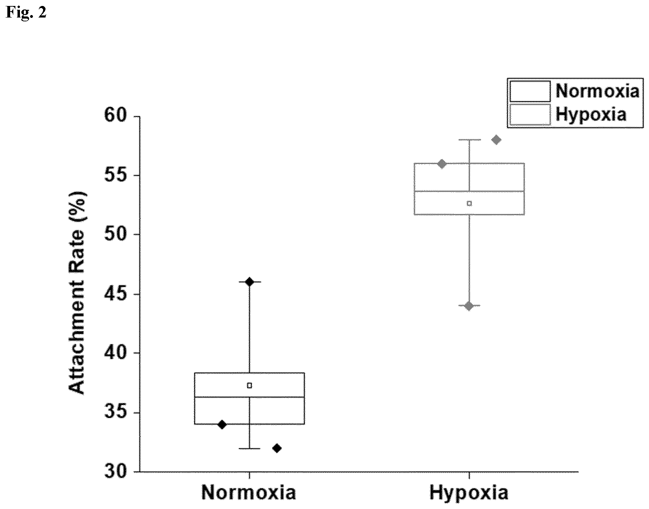

As in Example 3 above, the dermal papilla tissue (DP) isolated from the scalp was cultured under hypoxic conditions (5 % CO₂: 2 % O₂) and normoxic conditions (5 % CO₂: 21 % O₂), and then the level of attachment of the dermal papilla cells (DPC) to the culture plate was compared at days 5-10, and the results are shown in Fig. 2.

As shown in Fig. 2, as a result of comparing the attachment rates on the 6th day after culture, it was confirmed that the number of dermal papilla cells attached to the culture plate was significantly increased when cultured under hypoxic conditions compared to when cultured under normoxic conditions. This means that the period of establishing (harvesting) primary cells of the dermal papilla cells, which takes 12 days or more on average under normoxic conditions (an oxygen saturation of 20%), is shortened by an average of 5 days, and the additional production process can be shortened during subculture.

### [Experimental Example 2]

### Analysis of attachment mechanism of dermal papilla cells

As confirmed in Experimental Example 1 above, it can be seen that the level of attachment to the culture plate of the dermal papilla cells isolated and cultured according to the present invention is increased. Therefore, in order to confirm the mechanism by which the dermal papilla cells are attached to the culture plate, the following was performed.

### 2-1. Nucleotide sequence (NGS) and ontology analysis

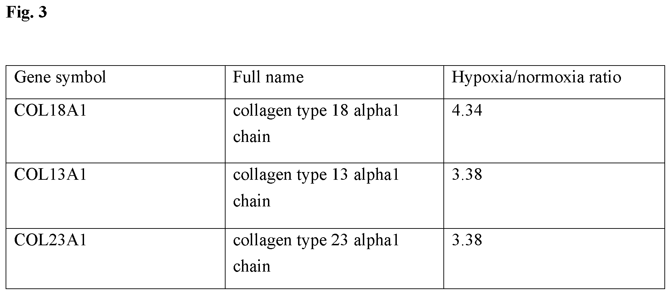

The nucleotide sequence and ontology analysis of the genes of the dermal papilla cells isolated and cultured under hypoxic conditions according to the present invention were performed. As a result, as shown in Fig. 3, it was confirmed that hypoxic conditions affect extracellular matrix (ECM) proteins, and it was confirmed that the collagen expression levels of Col18A1, Col13A1, and Col23A1 among them were increased. The collagen affects the activity of dermal papilla cells (*see* TGF-β2 and collagen play pivotal roles in the spheroid formation and antiaging of human dermal papilla cells. Kim H, Choi N, Kim DY, Kim SY, Song SY, Sung JH. Aging (Albany NY). 2021 Aug 17; 13(16): 19978 19995.). Through this, it can be seen that the expression of collagen increased by hypoxic conditions affects the attachment of dermal papilla cells to the culture plate.

### 2-2. Analysis of mRNA expression of extracellular matrix and adhesion molecules

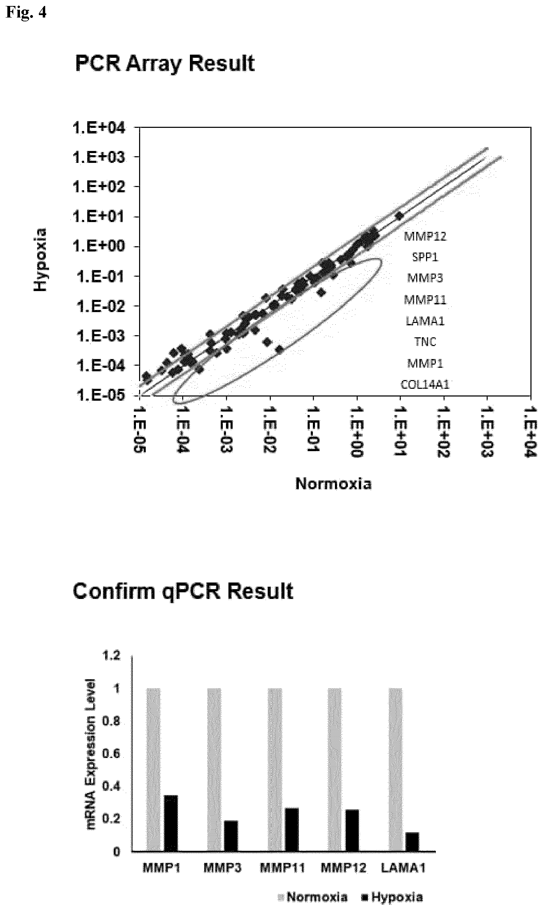

The mRNA expression levels of extracellular matrix and adhesion molecules of the dermal papilla cells isolated and cultured under hypoxic conditions according to the present invention were analyzed. As a result, as shown in Fig. 4, it was confirmed that the expression of MMP1, MMP3, MMP11, MMP12, and LAMA1 among various genes was reduced. In particular, MMP (matrix metalloproteinase) is an enzyme that degrades collagen. Through this, it can be seen that MMP expression reduced by hypoxic conditions inhibits collagen degradation and affects the attachment of dermal papilla cells to the culture plate.

### [Experimental Example 3]

### Evaluation of dermal papilla cell isolation and process shortening period

In order to evaluate the period of establishing (harvesting) the dermal papilla cells isolated and cultured according to the present invention, an experiment was performed as follows.

The dermal papilla tissue (DP) isolated from hair follicles was inoculated on a culture plate, cultured under hypoxic conditions (5 % CO₂: 2 % O₂) and normoxic conditions (5 % CO₂: 21 % O₂), and stored as a cell stock in the 3rd passage (filling). The period required was evaluated by dividing it as follows: the period of time it takes for attachment of 80 % or more of the total dermal papilla tissue (DP) inoculated per well in a 6 well plate is defined as 'isolation → attachment'; the period of time it takes until the 1st passage, when the cell density reaches 60 % or more of 1 well, is defined as 'isolation → 1st passage'; the period of time it takes until the 2nd passage, when the cell density reaches 80 % or more after the 1st passage, is defined as 'isolation → 2nd passage'; the period of time it takes until the 3rd passage, when the cell density reaches 80 % or more after the 2nd passage, is defined as 'isolation → 3rd passage'; and the period of time it takes to fill with stock after the 3rd passage is defined as 'isolation → filling'. The results are shown in Table 1 below.

**[Table 1]**

| Culture conditions | isolation→ attachment | isolation→ 1st passage | isolation→ 2nd passage | isolation→ 3rd passage | isolation→ filling |
|---|---|---|---|---|---|
| normoxic conditions | 12 days | 28 days | 33 days | 37 days | 41 days |
| hypoxic conditions | 7 days | 14 days | 18 days | 22 days | 26 days |

As shown in Table 1 above, as a result of measuring the period of time it takes for the dermal papilla tissue to be isolated and filled, it took 41 days when cultured under normoxic conditions, whereas it took 26 days when cultured under hypoxic conditions, confirming that it was shortened by about 15 days. In addition, the period of time it takes for dermal papilla cells from the isolated dermal papilla tissue to be attached was significantly shortened by 5 days when cultured under hypoxic conditions.

Therefore, it can be seen that in establishing (harvesting) dermal papilla cells, when the isolated dermal papilla tissue is cultured under hypoxic conditions, a significantly shorter period of time is required compared to when cultured under normoxic conditions, and the period until final filling is shortened by 15 days days or more.

### [Experimental Example 4]

### Evaluation of marker expression in dermal papilla cells

In order to confirm that the cells isolated and cultured according to the present invention were dermal papilla cells, they were analyzed by flow cytometry using an antibody that binds to the surface antigen of dermal papilla cells.

Dermal papilla cells of each passage dissociated in the culture solution were washed twice with DPBS. The cells were resuspended with 500 µl of cell staining buffer (1 % BSA in DPBS) per 1 × 10⁶ cells, and then the cell suspension was dispensed at 100 µl per a test group tube. 1 µl of the antibody corresponding to each test group was dispensed, and then light was blocked and reacted at 4 °C for 15 minutes. The test group stained with the antibody was washed once with DPBS, and then the cells were resuspended in 100 µl of DPBS and analyzed using a flow cytometer, and the results are shown in Table 2 below.

The antibodies corresponding to each test group are as follows: heterogenic antibody control group (APC Mouse IgG1_κ Isotype Ctrl Antibody), expression evaluation staining group (for identification of dermal papilla cells: APC anti-human CD34 Antibody, APC anti-human CD45 Antibody), purity evaluation staining group (APC anti-human CD44 Antibody, APC anti-human CD90 Antibody).

In a gate where the distribution rate of the control group was less than 0.1 %, if the distribution rate of the expression evaluation staining group for identification was 90 % or more and the distribution rate of the purity evaluation staining group was less than 10%, it was evaluated as suitable.

**[Table 2]**

| | Antibody | P2 | P3 | P4 | P5 |
|---|---|---|---|---|---|
| normoxic conditions | | | | | |
| purity (%) | CD34 | 0.00 | 0.19 | 0.19 | 0.37 |
| | CD45 | 0.00 | 0.02 | 0.03 | 0.00 |
| identification | CD44 | 99.97 | 100.00 | 100.00 | 99.99 |

| (%) | CD90 | 99.58 | 99.97 | 100.00 | 99.99 |
|---|---|---|---|---|---|
| hypoxic conditions | | | | | |
| purity (%) | CD34 | 0.07 | 0.00 | 0.06 | 0.25 |
| | CD45 | 0.03 | 0.00 | 0.01 | 0.00 |
| identification (%) | CD44 | 99.90 | 99.98 | 100.00 | 99.99 |
| | CD90 | 97.93 | 99.95 | 99.97 | 100.00 |

As shown in Table 2 above, as a result of evaluating marker expression in the dermal papilla cells of each passage (p), the expression of antibodies for purity was shown to be less than 1 % in the dermal papilla cells of all passages, and the expression of antibodies for identification was shown to be 90 % or more. Therefore, it was confirmed that the cells isolated and cultured according to the present invention were dermal papilla cells.

### [Experimental Example 5]

### Evaluation of immune response activity gene expression in dermal papilla cells

In order to evaluate the expression level of genes related to immune response activity in cells isolated and cultured according to the present invention, an experiment was performed as follows.

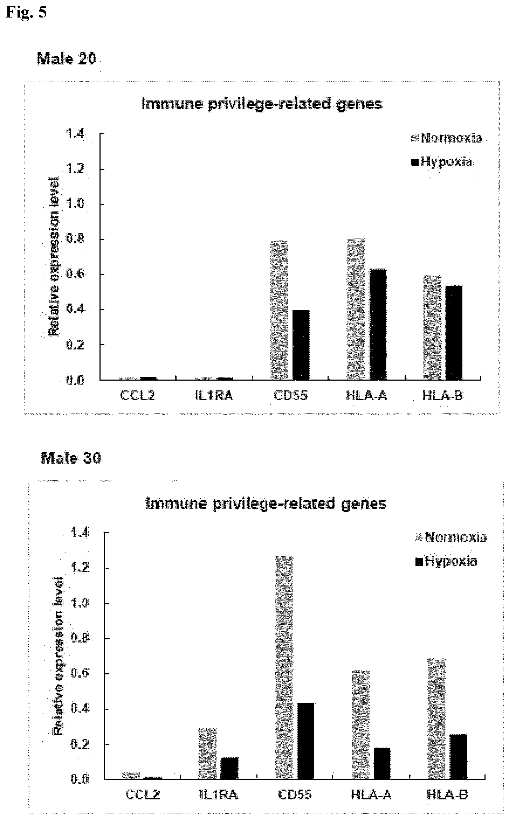

Dermal papilla cells were isolated from hair follicles (dermal papilla tissue) of male donors in their 20s and 30s and cultured under hypoxic conditions (5 % CO₂: 2 % O₂) and normoxic conditions (5 % CO₂: 20% O₂). After the 3rd passage, RNA from the cultured dermal papilla cells was extracted, and the mRNA expression levels of genes related to immune response activity of T cells were analyzed, and the results are shown in Fig. 5. Skin fibroblasts (mRAN expression level: 1) were used as a control group.

As shown in Fig. 5, as a result of analyzing the expression level of genes related to immune response activity through RNA of dermal papilla cells, it was confirmed that when the dermal papilla cells were isolated and cultured under hypoxic conditions, the expression of HLA-A, HLA-B, CD55, IL-1RA, and CCL2 was significantly reduced compared to normoxic conditions. In particular, the expression of HLA-A, HLA-B, and CD55 was significantly reduced. Through this, it can be seen that dermal papilla cells isolated and cultured under hypoxic conditions can reduce the immune response activity by T cells.

Therefore, it can be seen that when the dermal papilla cells are isolated and cultured under hypoxic conditions according to the present invention, the immune privilege, that is, the immune modulation function, of the dermal papilla cells is enhanced, and thus the immune response that may occur during the allogeneic transplantation of the dermal papilla cells can be reduced. Ultimately, it can be seen that the dermal papilla cells isolated and cultured according to the present invention have immunocompatibility.

## Claims

1. A method for isolating and culturing dermal papilla cells, comprising culturing dermal papilla tissue isolated from the scalp under hypoxic conditions and attaching the dermal papilla cells to a culture plate.

2. The method for isolating and culturing dermal papilla cells according to claim 1, wherein the hypoxic conditions have an oxygen saturation of 0.5 to 5%.

3. The method for isolating and culturing dermal papilla cells according to claim 1, wherein the hypoxic conditions have an oxygen saturation of 2%.

4. The method for isolating and culturing dermal papilla cells according to claim 1, wherein the period required for attaching the dermal papilla cells to the culture plate is shortened by 5 days or more compared to culturing the dermal papilla tissue under normoxic conditions.

5. The method for isolating and culturing dermal papilla cells according to claim 1, wherein the dermal papilla cells are immunocompatible dermal papilla cells.

6. The method for isolating and culturing dermal papilla cells according to claim 5, wherein the immunocompatible dermal papilla cells inhibit the expression of any one or more mRNAs selected from the group consisting of HLA-A, HLA-B, CD55, IL-1RA, and CCL2.

7. Immunocompatible dermal papilla cells according to the isolation and culture method according to claim 1.
